# EUROPEAN PATENT SPECIFICATION

(11) **EP 2 197 905 B1**
(45) Date of publication and mention of the grant of the patent: **26.12.2012**
(21) Application number: 08789669.2
(22) Date of filing: 02.09.2008
(51) Int. Cl.: C07K 14/78, C07K 14/435

(54) **MULTI-BLOCK COPOLYMERS**
MULTI-BLOCK-COPOLYMERE
COPOLYMÈRES À BLOCS MULTIPLES

(30) Priority: 07.09.2007 EP 07115943
(43) Date of publication of application: 23.06.2010
(73) Proprietor: Stichting Dutch Polymer Institute, 5612 AB Eindhoven (NL)
(72) Inventor: DE WOLF, Frederik Anton, NL-6708 PD Wageningen (NL); COHEN STUART, Martien, NL-5656AE Eindhoven (NL); EGGINK, Gerrit, NL-6708 PD Wageningen (NL); WERTEN, Marc Willem Theodoor, NL-6708 PD Wageningen (NL); MARTENS, Aernout Anders, NL-6703 HB Wageningen (NL)
(74) Representative: 't Jong, Bastiaan Jacob
(86) International application number: PCT/IB2008/053539
(87) International publication number: WO 2009/031095

(56) References cited:
- WO-A2-2005/094911
- CAPPELLO J ET AL: "GENETIC ENGINEERING OF STRUCTURAL PROTEIN POLYMERS" BIOTECHNOLOGY PROGRESS, AMERICAN INSTITUTE OF CHEMICAL ENGINEERS, US, vol. 6, no. 3, 1 May 1990 (1990-05-01), pages 198-202, XP000654980 ISSN: 8756-7938
- DINERMAN A A ET AL: "Swelling behavior of a genetically engineered silk-elastinlike protein polymer hydrogel" BIOMATERIALS, ELSEVIER SCIENCE PUBLISHERS BV., BARKING, GB, vol. 23, no. 21, 1 November 2002 (2002-11-01), pages 4203-4210, XP004373432 ISSN: 0142-9612
- VAN HEST J C ET AL: "Protein-based materials, toward a new level of structural control." CHEMICAL COMMUNICATIONS (CAMBRIDGE, ENGLAND) 7 OCT 2001, no. 19, 7 October 2001 (2001-10-07), pages 1897-1904, XP002475120 ISSN: 1359-7345
- LOWIK DENNIS W P M ET AL: "Synthesis of bio-inspired hybrid polymers using peptide synthesis and protein engineering" PEPTIDE HYBRID POLYMERS SERIES : ADVANCES IN POLYMER SCIENCE, 2006, pages 19-52, XP002516881 ISSN: 0065-3195 ISBN: 3-504-32567-0

## Description

### FIELD OF THE INVENTION

The invention relates to multi-block copolymers, more in particular to pH switchable multi-block copolymers, even more in particular to tri-block copolymers, still more in particular to tri-block copolymers forming supramolecular nano fibers.

### BACKGROUND OF THE INVENTION

Self-assembly and spontaneous formation of complex well-defined structures driven by non-covalent interactions has become a predominant theme in chemistry, material science and nanotechnology. Much of the inspiration for synthetic building blocks, supramolecular structures and nanostructured materials comes from biology. For example, one of the most successful 'work horses' of supramolecular chemistry, the ureidopyrimidinone quadruple hydrogen bonding group was inspired by the nucleotide bases. Inside and between natural proteins a subtle interplay between many different non-covalent interactions determines an enormous variety of well-defined molecular structures and aggregates. It seems attractive to utilize naturally occurring peptide sequences, with known non-covalent interactions, to create new supramolecular structures. Because of recent developments in molecular biology, it is now feasible to design and produce new peptide copolymers by biological expression of a designed DNA template. The peptide copolymers can be regarded as intermediates between synthetic polymers and natural proteins. The amino-acid sequence of these peptide copolymers can be tailored to make peptide copolymers with varying complexity. The peptide copolymers can be regarded as interesting models for natural proteins. They also prove to be useful as copolymers with perfectly defined length and a monomer sequence.

Usually block-copolymers are built from blocks of homopolymers. However, using the natural protein production biomachinery, blocks can be made consisting of sequences of different monomers (aminoacids), enlarging the repertoir of functions, forms and physical behavior that blocks can display. Most amino acid sequences used in blocks have their origin in natural sequences. Natural sequences with certain behavior are rationally redesigned and repeated to reach the desired length of a block. Examples of such natural sequences with interesting behavior are the glycine, alanine-rich repeats in silk proteins and collagen molecules, rich in proline

Silk-like blocks including an amino acid sequence (GAGAGAGX)ₙ, where G is glycine, A is alanine and X is either glutamic acid (E, anionic or partially anionic above pH of about 3-5), or lysine (K, cationic below pH 10) are reported in the literature. A silk-like block with X being E has been descried in the literature. Similarly, a silk-like block with X being K² and a silk-like block with X being alternatingly Y,E,H and K has already been described ^{3,4}. The silk-like block containing glutamic acid (E) is negatively charged and will form supramolecular structures only under acidic conditions. For some applications it may be necessary to form the structures at neutral pH or under basic conditions.

A polymer with (GAGAGAGE)ₙ mid block and chemically attached polydisperse PEG chains on either side has been described ^{5,6}. Chemical synthesis of the polymer with (GAGAGAGE)ₙ mid block and chemically attached polydisperse PEG chains on either side may involve many processing steps and may be cumbersome.

EP 1790657-A1 discloses pH-switchable transmembrane peptides as well as complexes containing them and their use as stimulators of membrane fusion. The peptides of the alleged invention are particularly useful for transfecting therapeutically active substances in eukaryotic cells. They describe peptides having an amino acid sequence of the formula (A)ₓ(B)y(C)_{z}(B)_{y}(A)ₓ wherein A is an essentially hydrophilic amino acid, preferably glycine (G), lysine (K) or arginine (R), particularly preferably histidine (H); X is 0 or 1; B is histidine (H); Y is an integer from 1 to 6; or Y is an integer from 0 to 5; when A is histidine (H) and X is the factor 1; C is a essentially hydrophobic amino acid, preferably selected from the group consisting of valine (V), leucine (L), Isoleucine (I), proline (P), glycine (G), cysteine (C), tryptophane (W), alanine (A), phenylalanine (F) and methionine (M); and Z is an integer from 7 to 30. The se peptides are very small and are used only as transmembrane peptides.

It is an objective of the invention to synthesize large multi-block copolymers which are capable of controlled self assembly and preferably can form supramolecular structures at any given pH.

### BRIEF SUMMARY OF THE INVENTION

According to a first aspect, the invention relates to a multi-block copolymer comprising at least one hydrophilic collagen-like block and at least one silk-like block, wherein the silk-like block comprises an amino acid sequence ((GA)ₘGX)ₙ, where G is glycine, A is alanine, m and n are at least 2 and X is an ionizable amino acid, and wherein the ionizable amino acid can be induced to bear either a positive charge or a negative charge.

According to a second aspect of the invention, a nano-wire structure comprises the above mentioned multi-block copolymer.

According to a third aspect, the multi-block copolymer is used for forming gels, cell culture or tissue engineering scaffolds, nano-wires, nano-wire templates, or it is used as an ingredient in composite materials.

### FIGURES

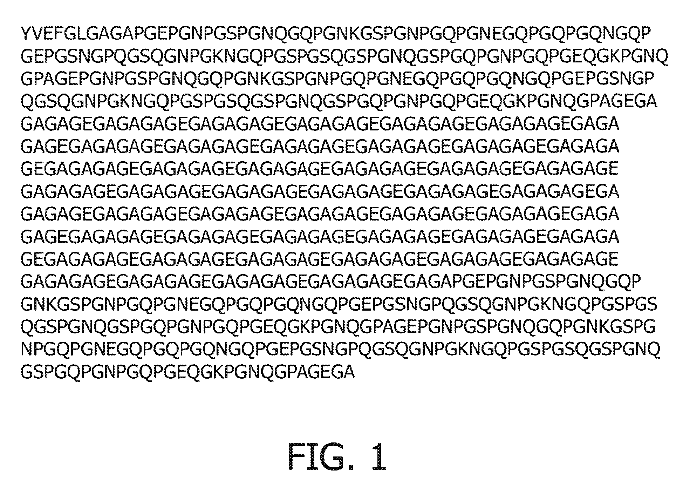

Figure 1 shows a complete sequence of CS^{E}S^{E}C in a single-letter amino acid code;

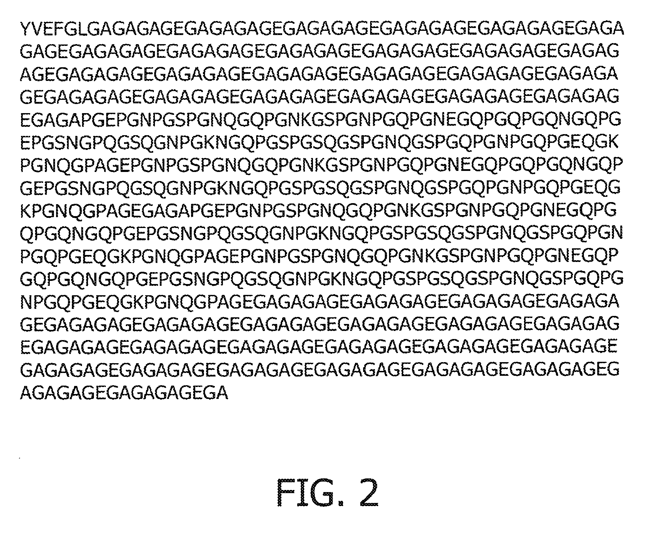

Figure 2 shows a complete sequence of S^{E}CCS^{E} in a single- letter amino acid code;

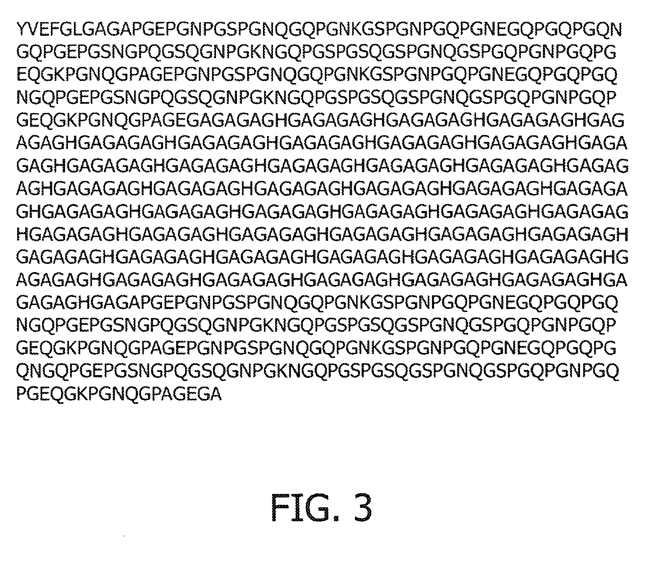

Figure 3 shows a complete sequence of CS^{H}S^{H}C in a single- letter amino acid code; and

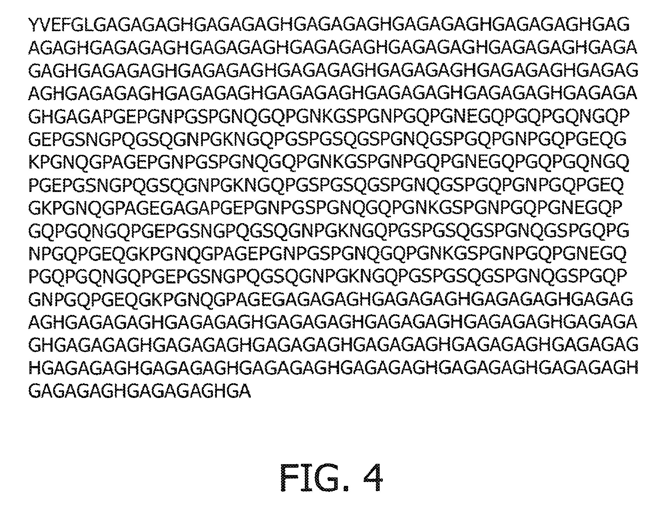

Figure 4 shows a complete sequence of S^{H}CCS^{H} in a single-letter amino acid code.

### DETAILED DESCRIPTION OF THE INVENTION

The invention relates to a multi-block copolymer comprising at least one hydrophilic collagen- like block and at least one silk- like block, wherein the silk- like block comprises an amino acid sequence ((GA)ₘGX)ₙ, where G is glycine, A is alanine, m and n are at least 2 and X is an ionizable amino acid, and wherein the ionizable amino acid can be induced to bear either a positive charge or a negative charge. The ionizable amino acid bearing a positive charge is histidine and the one bearing a negative charge is glutamic acid. Protonation of histidine occurs under acidic conditions i.e., at pH < 7.

Histidine has a low pKa and allows manipulating the charge at mild pH. Further, a molecule like (GAGAGAGH)ₙ, having the same charge molecules all over, makes the molecule soluble at neutral to acidic pH because of charge repulsion. The exact identity (equal structure) of all molecules of a single type and the pure enantiomeric structure allow better and faster folding into specific secondary and tertiary structures as well as self-assembly into crystalline supramolecular structures. The entire monomer sequence throughout the whole molecule is fully defined. All monomers are preferably L-a-amino acids. All individual blocks preferably have exactly the same structure and length in each single molecule. As a preferred feature of the invention, the sample is fully monodispersed. This allows specific and temporally stable structures such as discrete nanofibers with an extreme aspect ratio (520 nm x 5-20 µm) to self-assemble spontaneously (bottom-up structure formation), as triggered (controlled in a reversible way) by external stimuli like pH or temperature.

Having charged aminoacids in the ((GA)ₘGX)ₙ block allows to charge and discharge the block, using pH. In lowering the pH, a soluble, negatively charged block becomes uncharged, changes its conformation and self-assembles into supramolecular structures. Changing the pH back, charges the block and causes these structures to fall apart again. In the case of a positively charged block, the same effect is achieved with an opposite pH change. This effect makes the conformation of the block and the formed supramolecular structures pH switchable. Also, blocks with opposite charge at moderate pH will assemble with each other, forming two-component supramolecular structures. The collagen-like blocks screen the silk-like blocks from each other so that they form defined fibrils instead of precipitating into an aggregate.

The collagen blocks also stabilize the fibrils in an aqueous solution, allowing the fibrils to form a gel. The collagen-like blocks are preferably highly polar and randomly coiled blocks with relatively few charges. These blocks impart stability when mixing oppositely charged blocks. When the collagen-like block forms a middle block of the multi-block copolymer, it serves as a cross-linker between fibrils. These multi-block copolymers may be non toxic, non allergenic, animal free. No toxic substances are formed upon degradation of these molecules. These block copolymers can be produced relatively cheaply on a large scale.

The multi-block copolymer is preferably a tri-block copolymer. Tri-block copolymers comprise a silk-like block and a collagen-like block. The collagen-like block forms an outer block and the silk-like block formes a middle block or the collagen- like block forms the middle block and the silk-like block forms the outer block. The silk-like block may have glutamic acid. Then the tri-block copolymer is represented as S^{E}CCS^{E} and CS^{E}S^{E}C. The tri-block copolymer can also comprise a silk-like block with histidine and is then represented as S^{H}CCS^{H} and CS^{H}SH^{C}. S^{E} stands for a silk-like block with E, glutamic acid and S^{H} stands for a silk- like block with H, histidine. C stands for a collagen-like block.

H H The block copolymer with the positive charge at the ends (S^{H}CCS^{H}) is preferably combined with the block copolymer with the negative charge in the middle (CS^{E}S^{E}C) and the block copolymer with the negative charge at the ends S^{E}CCS^{E} is combined with the positive charge in the middle CS^{H}S^{H}C. When mixing these oppositely charged block copolymers with opposite architecture, the middle block of one block copolymer will associate with oppositely charged end blocks of the other at high concentrations, forcing a partial overlap of the blocks and thus resulting in a network formation. The two blocks S^{E} and S^{H} are water soluble as separate components but aggregate when mixed. They are temporarily stabilized during mixing by collagen-like blocks. Gel formation proceeds in a matter of minutes, allowing good mixing.

In the amino acid sequence ((GA)ₘ GX)ₙ, m is preferably in a range of 2 to 10 and n is preferably in a range of 3 to 100. If m is very large, the propensity to aggregate will increase. The charge density i.e., the force that is keeping the strands apart and soluble will decrease. To make a molecule soluble and also pH switchable, the amount of GA repeat and the amount of charge should be in balance. Further, if n is very small, the driving force for the aggregation / self-assembly will be small. The hydrophilic outer blocks will dominate, pulling the fibril apart, so the size of A and B blocks will be more in balance.. It is preferred that m is in a range from 2 to 5 and n is in a range from 25 to 60. We have found out that m equal to 3 and n equal to 48 gave good balance and therefore this combination is most preferred. It is preferred to make large molecules, so that the fibrils formed will reside in a deep energy minimum. When molecules of opposite architecture ABBA and BAAB are combined at high concentrations, the blocks might partially overlap serving as a cross-linking mechanism, which might enable to make macroscopic fibers. When producing macroscopic fibers, long molecules (802 amino acids) are preferable over short ones. Covalent bonds are stronger than intermolecular interactions. Large molecules give a larger amount of covalent bonds per unit weight of material. In addition to covalent bonds, intermolecular entanglements may occur in long molecules, which could be advantageous. Where long molecules secure the attachment of other molecules to the fiber matrix with the same amount of intermolecular bonds per molecule as do short molecules, long molecules will enable to span a larger distance with the same amount of intermolecular bonds per molecule than short molecules, especially in oriented stretched molecules such as in fibers. This may lead to more flexible (less brittle) fibers, which is advantageous.

The collagen block is preferably a non-assembling block. The non-assembling collagen block has unchanged behavior under a wide range of pH, temperature and osmotic pressure values. However, an assembling collagen may also be used for additional material properties, as it can add futher entanglements that will be beneficial to material properties of a fibre. It will be appreciated by the skilled person that, if an assembling collagen block is used, specific processing temperatures will be required.

According to another aspect of the invention, the invention relates to a nano-wire structure comprising the above mentioned block copolymers. These block copolymers are capable of forming reversible supramolecular self-assembling nano-sized tapes. These tapes possess an extreme aspect ratio. The silk-like blocks comprising glutamic acid are negatively charged and only capable of forming tapes at a low pH or when mixed with a positively charged, possibly conductive, poly electrolyte. Therefore, a positively charged counterpart is needed that will form tapes at a high pH or when mixed with a negatively charged, possibly conductive, poly electrolyte. This positively charged counterpart will also co-assemble with S^{E} at a neutral pH to form complex co-asseverate tapes. S^{H} forms such a positively charged counterpart. The nano-wire structures thus formed may be biocompatible. Insertion of biomimetic amino acid sequences elicits enhanced biocompatibility. For example the collagen-like mid block can bind human cells. The multi-block copolymers are non-immunogenic and non-allergenic, since silk and collagen are both beneficial in this respect. In principle, other structures like increased or decreased number of cell binding sites, increased, decreased or 'triggerable' biodegradability (enzymatic cleavage / protease recognition sites) bioactive sequences (growth factor- like, antimicrobial, etc.) can be easily inserted by genetic engineering into the random coil collagen-like block without disturbing the fibril-forming capacity of the silk-like block.

The invention will now be illustrated by the following non-limiting examples.

### Example 1: Production of template DNA coding for the S^{E} block

The first template block, coding for a negatively charged silk- like sequence (S^{E} block), was produced as follows. Double-stranded DNA was constructed by annealing of oligonucleotides: 
5’-
AATTCGGTCTCGGTGCTGGTGCTGGTGCTGGTGAGGGAGCCGGTGCTGGAGCCG
GCGAAGGTGCCTAAGCGGCCGC-3’ SEQ ID 1
 and

5’-
TCGAGCGGCCGCTTAGGCACCTTCGCCGGCTCCAGCACCGGCTCCCTCACCAGCA
CCAGCACCAGCACCGAGACCG-3’. SEQ ID 2

The double-stranded DNA was then ligated into an *EcoR*I/*Xho*I digested pMTL23 vector⁷, which had been modified to remove the *Bsa*I site normally present. The insert was elongated to hexamer-size (encoding 24 repeats of the amino acid sequence GAGAGAGE) by digestion with *Bsa*I/*Ban*I and directional ligation.

### Example 2: Production of template DNA coding for the S^{H} block

The second template block, coding for a positively charged silk- like sequence (S^{H} block), was produced similarly. Double-stranded DNA was constructed by annealing of oligonucleotides: 
5'- AATTCGGTCTCGGTGCTGGTGCTGGTGCTGGTCACGGAGCCGGTGCTGGAGCCGG
CCATGGTGCCTAAGCGGCCGC-3' SEQ ID 3
 and

5'-
TCGAGCGGCCGCTTAGGCACCATGGCCGGCTCCAGCACCGGCTCCGTGACCAGC
ACCAGCACCAGCACCGAGACCG-3'. SEQ ID 4

The double-stranded DNA was then ligated into the *EcoRI*/*XhoI* sites of the modified pMTL23 vector described above. The insert was elo ngated to hexamer-size (encoding 24 repeats of the amino acid sequence GAGAGAGE) by digestion with *Bsa*I/*Ban*I and directional ligation.

### Example 3: Production of template DNA coding for the Collagen like block (C-block)

The third template block, coding for a hydrophilic collagen-like sequence (C-block) was produced as follows. A double-stranded adapter was constructed by annealing of oligonucleotides:
5'-AATTCGGTCTCGGTGCTGGTGCACCCGGTGAGGGTGCCTAAGCGGCCGC-3' SEQ ID 5 and
5'-GCCAGAGCCACGACCACGTGGGCCACTCCCACGGATTCGCCGGCGAGCT-3'. SEO ID 6

The adapter was then inserted into the *EcoR*I/*Xho*I sites of the modified pMTL23 vector described above. The resulting vector was linearized with *Dra*III and dephosphorylated. The gene encoding the hydrophilic collagen-like sequence (P2) was cut from the previously described vector pMTL23-P2⁸ with *DraIII*/*Van911* and inserted into the linearised vector, creating the *C*-block template.

### Example 4: Connecting the template DNA coding for S^{H} and S^{E} to DNA coding for the collagen block

*Bsa*I and *Ban*I were used for digestion and directional ligation of the three template blocks, first into diblocks S^{E}C, CS^{E}, S^{H}C and CS^{H} and then into tetrablocks S^{E}CCS^{E}, CS^{E}S^{E}C, S^{H}CCS^{H} and CS^{H}S^{H}C. Finally, the tetrablocks were cloned into expression vector pPIC9 (Invitrogen) using *Eco*RI and *Not*I. The resulting vectors were linearized with *SalI* to promote homologous integration at the *his4* locus upon transformation of *Pichia pastoris* GS115 by electroporation, as described previously⁹

### Example 5 :Polymer production and purification

Polymer production¹⁰ was induced by switching from glycerol to 0.2 % (v/v) methanol as a carbon source, in fed-batch fermentations of *Pichia pastoris* in 2.5-liter Bioflo 3000 fermenters (New Brunswick Scientific). The pH was maintained at pH 5 for negatively charged polymers and at pH 3 for positively charged polymers. The polymers were produced and secreted into the fermentation medium, which was separated from the cells by 15 minutes centrifugation at 2000g and 4°C (in a Sorval centrifuge with a SLA 1500 rotor), followed by microfiltration of the supernatant. The polymers precipitated selectively from the fermentation supernatant by adding ammonium sulphate to a final concentration of 164 (g/kg) (30 % saturation), incubating for 30 min at 21 °C and centrifugating for 20 min. at 8000g and 4°C (Sorval, SLA1500). Pellets of negatively charged polymers were dissolved in 10 mM ammonia (pH9), while pellets of positively charged polymers were dissolved in 10 mM formic acid. The polymer pellets were dissolved in 20% of the original volume and the precipitation procedure was repeated once. The resuspended polymers were selectively precipitated by adding acetone to a final concentration of 80% (v/v). Resuspension and acetone precipitation was repeated once more, after which the pellets were resuspended in water and freeze-dried for storage. The salt-containing freeze-dried products were each resuspended in 100 ml 50 mM ammonia and dialyzed four times 18 h against 4 L of 10 mM ammonia, after which the polymers were freeze-dried again and used for experiments.

### References:

1. Krejchi, M.T. et al. Chemical Sequence Control Of Beta-Sheet Assembly In Macromolecular Crystals of Periodic Polypeptides. Science 265, 1427-1432 (1994).
2. Cantor, E.J. et al. Effects of amino acid side-chain volume on chain packing in genetically engineered periodic polypeptides. Journal Of Biochemistry 122, 217-225 (1997).
3. Topilina, N.I. et al. Bilayer fibril formation by genetically engineered polypeptides: Preparation and characterization. Biomacromolecules 7, 1104-111 (2006).
4. Higashiya, S., Topilina, N.I, Ngo, S.C., Zagorevskii, D. & Welch, J.T. Design and preparation of beta-sheet forming repetitive and block-copolymerized polypeptides. Biomacromolecules 8, 1487-1497 (2007).
5. Smeenk, J.M. et al. Controlled assembly of macromolecular beta-sheet fibrils. Angewandte Chemie-International Edition 44, 1968-1971 (2005).
6. Smeenk, J.M. et al. Fibril formation by triblock copolymers of silklike beta-sheet polypeptides and poly(ethylene glycol). Macromolecules 39, 2989-2997 (2006).
7. Chambers, S.P., Prior, S.E., Barstow, D.A. & Minton, N.P. The Pmtl Nic-Cloning Vectors .1. Improved Puc Polylinker Regions To Facilitate The Use Of Sonicated Dna For Nucleotide Sequencing. Gene 68, 139-149 (1988).
8. Werten, M.W.T., Wisselink, W.H., van den Bosch, T.J.J., de Brin, E.C. & de Wolf, F.A. Secreted production of a custom-designed, highly hydrophilic gelatin in Pichia pastoris. Protein Engineering 14, 447-454 (2001).
9. Werten, M.W.T., Van den Bosch, T.J., Wind, R.D., Mooibroek, H. & De Wolf, F.A. High-yield secretion of recombinant gelatins by Pichia pastoris. Yeast 15, 1087-1096 (1999).
10. Werten, M.W.T. & de Wolf, F.A. Reduced proteolysis of secreted gelatin and Yps1-mediated alpha-factor leader processing in a Pichia pastoris kex2 disruptant. Applied And Environmental Microbiology 71, 2310-2317 (2005).

## Claims

1. A multi-block copolymer comprising at least one hydrophilic collagen- like block and at least one silk- like block, wherein the silk- like block comprises an amino acid sequence ((GA)ₘGX)ₙ, where G is glycine, A is alanine, m and n are at least 2 and X is an ionizable amino acid, and wherein the ionizable amino acid can be induced to bear either a positive charge or a negative charge.

2. The copolymer of claim 1, wherein the ionizable amino acid bears a positive charge and is histidine.

3. The copolymer of claim 1, wherein the ionizable amino acid bears a negative charge and is glutamic acid.

4. The copolymer according to claim 1, which is a tri-block copolymer.

5. The copolymer according to any of claims 1 to 4, wherein the collagen- like block forms an outer block and the silk- like block forms a middle block.

6. The copolymer according to any of claims 1 to 4, wherein the collagen- like block forms the middle block and the silk-like block forms the outer block.

7. The copolymer according to any of claims 1 to 4, wherein m is in a range of 2- 10.

8. The copolymer according to any of claims 1 to 4, wherein n is in a range of 3- 100.

9. The copolymer according to any of claims 1 to 4, wherein m and n are in a range of 2- 10 and 3- 100.

10. The copolymer according to any of claims 1 to 4, wherein the collagen- like block is a non-assembling block.

11. A nano-wire structure comprising the block copolymer according to any of the claims 1 to 10.

12. Use of a multi-block copolymer according to any of claims 1 to 10 for forming gels, cell culture or tissue engineering scaffolds, nano-wires, nano-wire templates, or for use as an ingredient in composite materials.

## Patentansprüche

1. Mehrblock-Copolymer, umfassend wenigstens einen hydrophilen collagenartigen Block und wenigstens einen seideartigen Block, wobei der seideartige Block eine Aminosäuresequenz ((GA)ₘGX)ₙ umfasst, wobei G Glycin ist, A Alanin ist, m und n wenigstens 2 sind und X eine ionisierbare Aminosäure ist, und wobei die ionisierbare Aminosäure induziert werden kann, entweder eine positive Ladung oder eine negative Ladung zu tragen.

2. Copolymer gemäß Anspruch 1, wobei die ionisierbare Aminosäure eine positive Ladung trägt und Histidin ist.

3. Copolymer gemäß Anspruch 1, wobei die ionisierbare Aminosäure eine negative Ladung trägt und Glutaminsäure ist.

4. Copolymer gemäß Anspruch 1, das ein Triblock-Copolymer ist.

5. Copolymer gemäß einem der Ansprüche 1 bis 4, wobei der collagenartige Block einen äußeren Block bildet und der seideartige Block einen mittleren Block bildet.

6. Copolymer gemäß einem der Ansprüche 1 bis 4, wobei der collagenartige Block den mittleren Block bildet und der seideartige Block den äußeren Block bildet.

7. Copolymer gemäß einem der Ansprüche 1 bis 4, wobei m im Bereich von 2-10 liegt.

8. Copolymer gemäß einem der Ansprüche 1 bis 4, wobei n im Bereich von 3-100 liegt.

9. Copolymer gemäß einem der Ansprüche 1 bis 4, wobei m und n im Bereich von 2-10 und 3-100 liegen.

10. Copolymer gemäß einem der Ansprüche 1 bis 4, wobei der collagenartige Block ein nicht aufbauender Block ist.

11. Nanodrahtstruktur, umfassend das Blockcopolymer gemäß einem der Ansprüche 1 bis 10.

12. Verwendung eines Mehrblock-Copolymers gemäß einem der Ansprüche 1 bis 10 zum Herstellen von Gelen, Zellkultur- oder Gewebeengineering-Gerüsten, Nanodrähten, Nanodraht-Templaten oder für die Verwendung als Bestandteil in Verbundstoffmaterialien.

## Revendications

1. Copolymère à blocs multiples comprenant au moins un bloc analogue au collagène hydrophile et au moins un bloc analogue à la soie, le bloc analogue à la soie comprenant une séquence d'acides aminés ((GA)ₘ(GX)ₙ), G représentant glycine, A représentant alanine, m et n valant au moins 2, et X représentant un acide aminé ionisable, et l'acide aminé ionisable pouvant être induit pour porter soit une charge positive, soit une charge négative.

2. Copolymère selon la revendication 1, l'acide aminé ionisable portant une charge positive et étant l'histidine.

3. Copolymère selon la revendication 1, l'acide aminé ionisable portant une charge négative et étant l'acide glutamique.

4. Copolymère selon la revendication 1, ledit copolymère étant un copolymère à trois blocs.

5. Copolymère selon l'une quelconque des revendications 1 à 4, le bloc analogue au collagène formant un bloc externe et le bloc analogue à la soie formant un bloc central.

6. Copolymère selon l'une quelconque des revendications 1 à 4, le bloc analogue au collagène formant le bloc central et le bloc analogue à la soie formant le bloc externe.

7. Copolymère selon l'une quelconque des revendications 1 à 4, m se situant dans la plage de 2-10.

8. Copolymère selon l'une quelconque des revendications 1 à 4, n se situant dans la plage de 3-100.

9. Copolymère selon l'une quelconque des revendications 1 à 4, m et n se situant dans la plage de 2-10 et de 3-100.

10. Copolymère selon l'une quelconque des revendications 1 à 4, le bloc analogue au collagène étant un bloc de non-assemblage.

11. Structure de nanofils comprenant le copolymère à blocs selon l'une quelconque des revendications 1 à 10.

12. Utilisation d'un copolymère à blocs multiples selon l'une quelconque des revendications 1 à 10 pour former des gels, des échafaudages de culture cellulaire ou d'ingénierie tissulaire, des nanofils, des motifs de nanofils ou pour une utilisation comme ingrédient dans des matériaux composites.
